# EUROPÄISCHE PATENTSCHRIFT

(11) **EP 0 355 351 B1**
(45) Veröffentlichungstag und Bekanntmachung des Hinweises auf die Patenterteilung: **10.11.1993**
(21) Anmeldenummer: 89112405.9
(22) Anmeldetag: 07.07.1989
(51) Int. Cl.: C07C 209/26

(54) **Verfahren zur Herstellung von Aminen**
Process for the preparation of amines
Procédé pour la préparation d'amines

(30) Priorität: 20.07.1988 DE 3824519; 13.10.1988 DE 3834848
(43) Veröffentlichungstag der Anmeldung: 28.02.1990
(73) Patentinhaber: BAYER AG, 51368 Leverkusen (DE)
(72) Erfinder: Kiel, Wolfgang, Dr., D-5068 Odenthal (DE); Ziemann, Heinz, Dr., D-5653 Leichlingen 1 (DE)

(56) Entgegenhaltungen:
- EP-A- 0 014 998
- DE-A- 2 941 070
- HOUBEN-WEYL: "Methoden der Organischen Chemie", Band IV/1c, 1980, Seiten 127,128,239,240,436, Georg Thieme Verlag, Stuttgart, DE

## Beschreibung

Die Erfindung betrifft ein Verfahren zur Herstellung von Aminen, in denen mindestens ein Substituent aromatisch ist und ein- bis dreifach durch Halogen substituiert ist. Hierbei wird von Umsetzungsprodukten aus Oxoverbindungen und Aminen oder Ammoniak ausgegangen, die Halogensubstituenten im genannten Umfange tragen. Solche Umsetzungsprodukte werden katalytisch hydriert, wobei ein Ni-haltiger und/oder Co-haltiger Katalysator eingesetzt wird und in Gegenwart von organischen Schwefelverbindungen gearbeitet wird.

Die katalytische Hydrierung von Umsetzungsprodukten aus Oxoverbindungen und Aminen oder Ammoniak ist bekannt. Als Oxoverbindungen seien gemäß folgendem Formelschema Aldehyde und Ketone genannt: 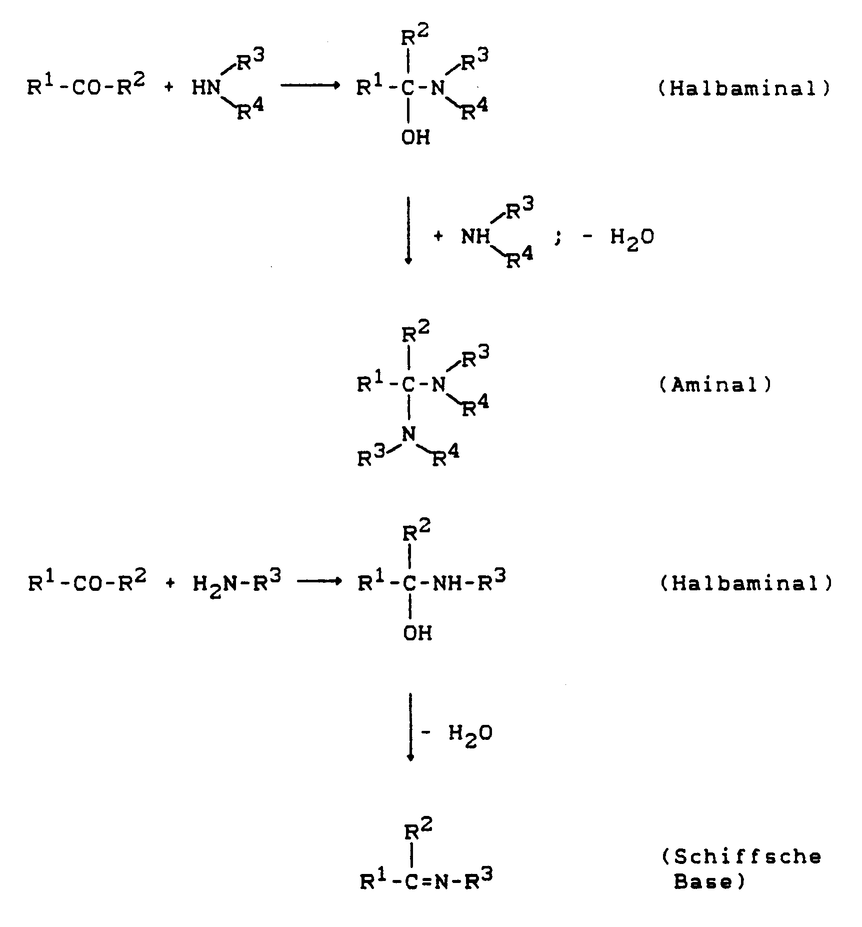
Aus einem Aldehyd oder Keton und einem primären oder sekundären Amin oder Ammoniak kann demnach zunächst ein Halbaminal gebildet werden, welches unter Austritt eines Moleküls Wasser mit einem weiteren Molekül des Amins oder mit Ammoniak zum Aminal weiterreagieren kann oder (nur bei primären Aminen oder Ammoniak) ebenfalls unter Austritt eines Moleküls Wasser zum Azomethin (Schiff'sche Base, Aldimin, Ketimin) reagieren kann. Aldimine als Umsetzungsprodukte von Aldehyden und Ammoniak können zusätzlich auf indirektem Weg durch partielle Hydrierung von Nitrilen erhalten werden. Alle genannten Umsetzungsprodukte (Halbaminal, Aminal bzw. Azomethin) können katalytisch zu den entsprechenden Aminen hydriert werden (Houben-Weyl, Methoden der Organischen Chemie, 4. Auflage, Band IV/1c (1980), S. 127/128, 239/240 u. 436). Solche katalytischen Hydrierungen sollen auch unter Erhaltung von Halogen möglich sein. Jedoch ist dieses Ergebnis auf die Benutzung von Palladium-Katalysatoren beschränkt, und es wird weiter darauf hingewiesen, daß man vorteilhafterweise mit desaktiviertem Katalysator und bei niederen Temperaturen arbeitet (Seite 240). Auch unter Verwendung anderer Katalysatoren, wie Platin oder Raney-Nickel soll Halogen erhalten bleiben (Houben-Weyl, loc. cit.). Die an den zitierten Stellen angegebenen speziellen Hydrierungen stellen jedoch keine systematische Untersuchung dar (Seite 436, Absatz 3) und zeigen zum Teil überaus mäßige Ausbeuten, wie im Falle des p-Chlorbenzyl-methyl-ketons, welches nur in 10 % der theoretischen Ausbeute in das zugehörige Amin übergeführt werden kann (Houben-Weyl, Seite 436 unten). Insbesondere bei der Herstellung stark basischer Amine muß mit Nebenprodukten gerechnet werden (Houben-Weyl, Seite 240, Absatz 2).

Es wurde nun überraschend gefunden, daß zur weitgehenden Erhaltung von aromatisch gebundenem Halogen bei der beschriebenen katalytischen Hydrierung an Ni-haltigen und/oder Co-haltigen Katalysatoren gearbeitet werden kann, wenn man gleichzeitig die weiter unten beschriebenen organischen Schwefelverbindungen einsetzt.

Die Erfindung betrifft demnach ein Verfahren zur Herstellung von Aminen der Formel 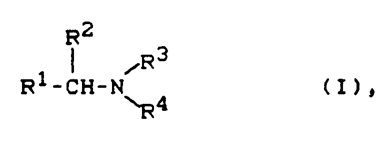
in der
- R¹, R² und R³: unabhängig voneinander Wasserstoff, geradkettiges oder verzweigtes C₁-C₁₂-Alkyl, C₃-C₈-Cycloalkyl, C₆-C₁₂-Aryl oder C₇-C₁₀-Aralkyl bedeuten und
- R⁴: für Wasserstoff, geradkettiges oder verzweigtes C₁-C₂₀-Alkyl, C₆-C₁₂-Aryl, C₃-C₈-Cycloalkyl oder C₇-C₁₀-Aralkyl steht,
wobei mindestens einer der Reste R¹ bis R⁴ Aryl oder Aralkyl darstellt, die im aromatischen Teil ein- bis dreifach durch Halogen substituiert sind,
durch katalytische Hydrierung von Umsetzungsprodukten aus Oxoverbindungen der Formel

R¹-CO-R² (II)

und Stickstoffverbindungen der Formel

R³-NH-R⁴ (III)

mit den obengenannten Bedeutungen für R¹ bis R⁴, welches dadurch gekennzeichnet ist, daß ein Ni-haltiger und/oder Co-haltiger Katalysator eingesetzt wird und in Gegenwart von organischen Schwefelverbindungen der Formel

R⁵-S(=O)ₙ-R⁶ (IV)

gearbeitet wird, in der
- R⁵ und R⁶: unabhängig voneinander geradkettiges oder verzweigtes C₁-C₁₂-Alkyl, Hydroxy-C₂-C₁₂-alkyl, Carboxy-C₁-C₁₂-alkyl oder Phenyl bedeuten und weiterhin
- R⁵ und R⁶: gemeinsam für -CH=CH-CH=CH-, -(CH₂)₄-, -(CH₂)₅-, -(CH₂)₂-S-(CH₂)₂- oder -(CH₂)₂-O-(CH₂)₂-stehen können,
- R⁶: zusätzlich Wasserstoff oder CO-C₁-C₁₂-alkyl bedeuten kann und
- n: den Wert 0 oder 1 annimmt.

Geradkettiges oder verzweigtes C₁-C₁₂-Alkyl ist beispielsweise Methyl, Ethyl, Propyl, Isopropyl, Butyl, Isobutyl, tert.-Butyl, Amyl, Hexyl, Octyl, Decyl oder Dodecyl; bevorzugt ist C₁-C₈-Alkyl, besonders bevorzugt C₁-C₄-Alkyl. R⁴ kann weiterhin auch bis zu 20 C-Atome haben und beispielsweise auch Palmityl, Stearyl oder Eicosyl sein. Hydroxy-alkyl trägt an beliebiger Stelle, bevorzugt in der ω-Position, eine Hydroxygruppe und kann weiterhin durch Ether-Sauerstoff in der Kohlenstoffkette unterbrochen sein. Carboxy-alkyl trägt an beliebiger Stelle, bevorzugt in α- oder ω-Position, eine Carboxylgruppe. Es gelten die gleichen Vorzugsbereiche wie für Alkyl.

C₃-C₈-Cycloalkyl ist beispielsweise Cyclopropyl, Cyclobutyl, Cyclopentyl, Cyclohexyl, Cycloheptyl oder Cyclooctyl, welches ein- oder zweifach durch Methyl und/oder Ethyl substituiert sein kann. Bevorzugtes Cycloalkyl ist Cyclopropyl, Cyclopentyl bzw. Cyclohexyl.

C₆-C₁₂-Aryl ist beispielsweise Phenyl, Naphthyl oder Biphenyl, bevorzugt Phenyl.

C₇-C₁₀-Aralkyl ist beispielsweise Benzyl, Phenylethyl oder Phenylpropyl, bevorzugt Benzyl und Phenylethyl.

Mindestens einer der Reste in den Umsetzungsprodukten von Oxoverbindungen mit den Stickstoffverbindungen ist Aryl oder Aralkyl und im aromatischen Teil ein- bis dreifach durch Halogen, wie Fluor, Chlor oder Brom, bevorzugt Fluor oder Chlor, besonders bevorzugt Chlor, substituiert. Für den Fall der mehrfachen Substitution kann es sich auch um eine Substitution durch verschiedene Halogenatome handeln. In bevorzugter Weise liegt eine ein- oder zweifache Substitution durch Halogen, in besonders bevorzugter Weise eine einfache Substitution durch Halogen vor.

Alle aromatischen Teile der Reste können weiterhin eine oder zwei Methyl- oder Ethylgruppen, Methoxy- oder Ethoxygruppen oder C₁-C₄-Dialkylaminogruppen tragen.

In bevorzugter Weise werden Oxoverbindungen der Formel

R¹¹-CO-R¹² (V)

eingesetzt, in der
- R¹¹: Wasserstoff, geradkettiges oder verzweigtes C₁-C₁₂-Alkyl, C₃-C₆-Cycloalkyl, Phenyl, Benzyl oder Phenyl-ethyl bedeutet und
- R¹²: für Wasserstoff, geradkettiges oder verzweigtes C₁-C₄-Alkyl oder Phenyl steht.

In besonders bevorzugter Weise werden Oxoverbindungen der Formel

R²¹-CO-R²² (VI)

eingesetzt, in der
- R²¹: Wasserstoff, geradkettiges oder verzweigtes C₁-C₄-Alkyl, C₅-C₆-Cycloalkyl, Phenyl, Benzyl oder Phenyl-ethyl bedeutet und
- R²²: für Wasserstoff, Methyl oder Ethyl steht.

In bevorzugter Weise werden weiterhin Stickstoffverbindungen der Formel

R¹³-NH-R¹⁴ (VII)

eingesetzt, in der
- R¹³: Wasserstoff, geradkettiges oder verzweigtes C₁-C₈-Alkyl, C₃-C₆-Cycloalkyl, Phenyl oder Benzyl bedeutet und
- R¹⁴: für Wasserstoff, geradkettiges oder verzweigtes C₁-C₄-Alkyl oder Benzyl steht.

In besonders bevorzugter Form werden Stickstoffverbindungen der Formel

R²³-NH-R²⁴ (VIII)

eingesetzt, in der
- R²³: Wasserstoff, geradkettiges oder verzweigtes C₁-C₄-Alkyl, C₅-C₆-Cycloalkyl, Phenyl oder Benzyl bedeutet und
- R²⁴: für Wasserstoff, Methyl oder Ethyl steht.

In erfindungsgemäßer Weise werden auch die bevorzugten bzw. besonders, bevorzugten Oxoverbindungen bzw. Stickstoffverbindung so kombiniert, daß in ihren Umsetzungsprodukten mindestens einer der darin genannten Reste Aryl oder Aralkyl ist und im aromatischen Teil ein- bis dreifach durch Halogen substituiert ist.

Das erfindungsgemäße Verfahren wird in Gegenwart einer oder mehrerer organischer Schwefelverbindungen der Formel (IV) durchgeführt. Beispiele für solche Verbindungen sind Bis-(2-hydroxyethyl)-sulfid, Bis-(2-hydroxypropyl)sulfid, Thiodiessigsäure, Thiodipropionsäure und andere ähnlich gebaute Verbindungen, ihre Alkalimetallsalze und ihre niederen Ester (beispielsweise ihre Dimethylester), Thioanisol, Diphenylsulfid, Dithian, Thioxan, Thiophen, Dimethylsulfoxid, Methylethylsulfoxid, Diethylsulfoxid. Die organische Schwefelverbindung wird in einer Menge von 0,002-0,5 Gew.-Teilen, bevorzugt 0,01-0,25 Gew.-Teilen, je Gew.-Teil Katalysator, eingesetzt. In bevorzugter Weise wird eine organische Schwefelverbindung der Formel

R¹⁵-S(=O)ₙ-R¹⁶ (IX)

eingesetzt, in der
- R¹⁵ und R¹⁶: unabhängig voneinander geradkettiges oder verzweigtes C₁-C₁₂-Alkyl, Hydroxy-C₂-C₁₂-alkyl oder Carboxy-C₁-C₁₂-alkyl bedeuten, wobei
- R¹⁶: zusätzlich CO-C₁-C₆-alkyl bedeuten kann und
- n: den Wert 0 oder 1 annimmt.

In besonders bevorzugter Weise wird Bis-(2-hydroxyethyl)-sulfid eingesetzt.

Der Zusatz der organischen Schwefelverbindung erfolgt gemeinsam mit dem Katalysator, vor der Zugabe oder nach der Zugabe des Katalysators. Wird der Katalysator mehrfach wiederverwendet, braucht die organische Schwefelverbindung im allgemeinen nur beim ersten Einsatz dem Katalysator bzw. dem Reaktionsansatz zugesetzt zu werden. Der Katalysator behält dann auch nach vielfach wiederholter Verwendung oder bei einer kontinuierlichen Verfahrensweise über lange Zeit seine hohe spezifische Aktivität bei gleichbleibend hoher Ausbeute bei. Eine Nachdosierung der organischen Schwefelverbindung ist. dennoch möglich, aber in der Regel nur erforderlich, wenn frischer Katalysator für etwa verbrauchten oder ausgeschleusten Katalysator zudosiert wird.

Als Hydrierkatalysatoren werden erfindungsgemäß Ni-haltige und/oder Co-haltige, wie Ni oder Co auf Trägern, Ni oder Co in Form von elementarem Ni(Co)-Schwamm, Ni-Oxid, Co-Oxid, Raney-Nickel, Raney-Kobalt oder andere eingesetzt. Träger sind beispielsweise SiO₂, Al₂O₃, Bims, Kohle und andere dem Fachmann bekannte Träger. In bevorzugter Weise werden jedoch Raney-Katalysatoren, wie Raney-Nickel, Raney-Kobalt, Raney-Nickel-Eisen, Raney-Nickel-Kobalt oder Raney-Nickel-Eisen-Kobalt in wasserfreier oder auch wasserfeuchter oder Lösungsmittelfeuchter Form eingesetzt. Die Ni- und Co-haltigen Katalysatoren können auch gemeinsam eingesetzt werden.

Der Ni-haltige und/oder Co-haltige Katalysator wird in einer Menge von 1-25 Gew.-%, bevorzugt 2,5-12,5 Gew.-%, bezogen auf das zu hydrierende Substrat, eingesetzt.

In besonders bevorzugter Weise werden Ni-haltige Raney-Katalysatoren eingesetzt.

Als Reaktionsmedium können Alkohole, wie Methanol, Ethanol, Isopropanol, Butanol, aliphatische oder aromatische Kohlenwasserstoffe, wie Toluol, Xylol, Cyclohexan, Isooctan und ähnliche, Ether, wie Tetrahydrofuran, Dioxan oder Methyl-tert.-butylether, Ester wie Ethylacetat und schließlich das Reaktionsprodukt selbst, sofern es bei der Reaktionstemperatur flüssig ist, eingesetzt werden. Ein Gehalt an Wasser (z.B. bis zu 20 Gew.-% des gesamten Reaktionsmediums) stört nicht, insbesondere, wenn das Reaktionsmedium mit Wasser mischbar ist.

Die Hydrierung wird bei 30-250°C, bevorzugt bei 50-150°C, und einem H₂-Druck von 5-200 bar, bevorzugt 10-150 bar, durchgeführt.

Im allgemeinen wird das erfindungsgemäße Verfahren so durchgeführt, daß man den Ausgangsstoff (Halbaminal, Aminal oder Azomethin), das Reaktionsmedium, den Katalysator und die organische Schwefelverbindung in einem Hydrierautoklaven vorlegt und nach Verschließen des Reaktors die Luft mit Stickstoff und anschließend den Stickstoff mit Wasserstoff verdrängt. Nach beendeter Reaktion wird das Reaktionsgefäß zunächst entspannt und entleert; der Katalysator wird abfiltriert und kann ohne neuen Zusatz von organischer Schwefelverbindungen wiederverwendet werden. Im übrigen erfolgt die Aufarbeitung in fachmännischer Weise.

Das Verfahren kann sowohl diskontinuierlich als auch kontinuierlich, beispielsweise in einem Druckrohr mit anschließendem Abscheider und Druckentspannung, durchgeführt werden.

Das Verfahren kann weiterhin sowohl auf die reinen genannten Umsetzungsprodukte als auch auf Reaktionsgemische, in denen Halbaminale, Aminale oder Azomethine als solche Umsetzungsprodukte hergestellt werden, angewandt werden.

In bevorzugter Weise werden Reaktionsgemische in denen solche Umsetzungsprodukte hergestellt werden, angewandt, wobei die entstehenen Umsetzungsprodukte simultan in erfindungsgemäßer Weise hydriert werden.

In weiterhin bevorzugter Weise werden Reaktionsgemische eingesetzt, in denen Aldimine der Formel

R³¹-CH=NH (X),

in der R³¹ die oben genannte Bedeutung hat,
durch partielle Hydrierung aus den zugrundeliegenen Nitrilen hergestellt werden, wobei die Aldimine simultan in erfindungsgemäßer Weise weiter hydriert werden. Die partielle Hydrierung der Nitrile wird ebenfalls durch den erfindungsgemäß eingesetzten Ni-haltigen und/oder Co-haltigen Katalysator in Gegenwart der organischen Schwefelverbindungen der Formel (IV) durchgeführt.

Es ist überraschend, daß durch die Mitverwendung der organischen Schwefelverbindungen als Modifizierungsmittel, für die in anderen Fällen eine Desaktivierung beobachtet wird, die beschriebene Hydrierung selektiver, reproduzierbar und vor allem vollständiger abläuft als ohne diese Zugabe; die Hydrieraktivität bleibt demnach vollständig erhalten. Die Katalysatoren haben eine hohe Standzeit. Eine Schädigung der Ni-haltigen und/oder Co-haltigen Katalysatoren durch unerwünschte Nebenprodukte wird nicht beobachtet, wodurch die beschriebene häufige Wiederverwendung möglich wird. Weiterhin wird die Anwendung sehr niedriger Reaktionstemperaturen, die extrem lange Reaktionszeiten erforderlich machen, vermieden. Die weitgehende Vermeidung von unerwünschten Nebenprodukten erhöht nicht nur die Ausbeute, sondern vereinfacht auch etwa nachgeschaltete Reinigungsoperationen.

### Beispiele (die Beispiele sind nicht optimiert; eine weitere Steigerung der Ausbeuten und Selektivitäten ist daher denkbar und wahrscheinlich)

### Beispiel 1: α-(p-Chlorphenyl)-ethylamin

In einem 3-Liter-Rührautoklaven wurden 385 g (2,5 Mol) p-Chloracetophenon, 1125 ml Methanol, 3,8 g Bis-(2-hydroxyethyl)-sulfid sowie 12,5 g Ammoniumacetat und 25 g Raney-Nickel vorgelegt. Nach Verdrängen der Luft mit Stickstoff und Zugabe von 750 ml flüssigem Ammoniak wurde Wasserstoff bis zu einem Druck von 80 bar aufgedrückt und unter Rühren auf 120°C geheizt. Nach Einstellung dieser Temperatur wurde der Druck durch weitere Zufuhr von Wasserstoff auf 120 bar erhöht und der Verbrauch kompensiert.

Nachdem die Wasserstoffaufnahme in etwa 2 Stunden beendet war, wurde noch eine Stunde unter den oben angegebenen Reaktionsbedingungen weiter gerührt, anschließend auf Raumtemperatur abgekühlt und auf Normaldruck entspannt.

Der Katalysator wurde abfiltriert und das Methanol abdestilliert. Als Rückstand erhielt man 388 g Rohprodukt, das nach gaschromatographischer Analyse
90% α-(p-Chlorphenyl)-ethylamin
5% α-Phenethylamin
4,5% α-(p-Chlorphenyl)-ethanol und
0,5% p-Chloracetophenon (Edukt)
enthielt.

Zur Reinigung wurde das Rohprodukt in 400 ml Toluol gelöst und mit 100 ml 20%iger Natronlauge gewaschen. Die toluolische Lösung wurde eingeengt und der Rückstand über eine Füllkörperkolonne destilliert. Man erhielt 316.4 g (74% der theoretischen Ausbeute) des gewünschten α-(p-Chlorphenyl)ethylamin als Hauptfraktion mit einem Siedepunkt von 115-117°C/25 mbar und einer Reinheit von 98,7%.

### Beispiel 2: α-(p-Chlorphenyl)-ethylamin

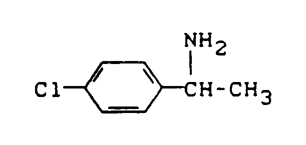

Die unter Beispiel 1 beschriebene reduktive Aminierung wurde wiederholt, wobei anstelle von Bis-(2-hydroxyethyl)-sulfid Dimethylsulfoxid in gleicher Menge eingesetzt wurde.

Nach Abtrennen des Katalysators und des Lösemittels wurden 386 g des rohen Umsetzungsproduktes erhalten, das nach gaschromatographischer Analyse
88,5% α-(p-Chlorphenyl)-ethylamin
5,5% α-Phenethylamin
4,5% α-(p-Chlorphenyl)-ethanol
1,0% p-Chloracetophenon (Edukt)
enthielt.

### Beispiel 3 (zum Vergleich):

Führte man die in den Beispielen 1 und 2 beschriebene reduktive Aminierung ohne Zusatz einer der Schwefelverbindungen durch, so erhielt man nach Abtrennen des Katalysators und des Lösemittels 384 g eines Rohproduktes, das nach gaschromatographischer Analyse
69% α-(p-Chlorphenyl)-ethylamin
26% α-Phenethylamin und
4% α-(p-Chlorphenyl)-ethanol
enthielt.

### Beispiel 4: N-Methyl-α-(p-chlorphenyl)-ethylamin

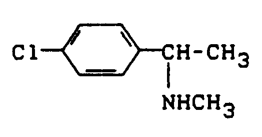

In einem 1,3-l-Rührautoklaven wurden 154 g p-Chloracetophenon, 450 ml Methanol, 20 g Raney-Nickel und 2 g Bis-(2-hydroxyethyl)-sulfid vorgelegt. Nach Verdrängen der Luft mit Stickstoff und Zugabe von 150 ml flüssigem Methylamin wurde Wasserstoff bis zu einem Druck von 80 bar aufgedrückt und unter Rühren auf 100°C geheizt. Durch weitere Zufuhr von Wasserstoff wurde der Druck auf 140 bar eingestellt und der Verbrauch kompensiert. Die Temperatur wurde noch bis 1 Stunde nach dem Ende der H₂-Aufnahme bei 100°C gehalten.

Nach dem Abkühlen und Entspannen wurde der Katalysator abfiltriert und die Reaktionslösung eingedampft.

Es wurden 170 g Rohprodukt erhalten, das nach gaschromatographischer Analyse
86% N-Methyl-α-(p-chlorphenyl)-ethylamin
5,5% α-(p-Chlorphenyl)-ethanol
6,5% N-Methyl-α-phenethylamin
2% nicht identifizierte Nebenprodukte
enthielt.

### Beispiel 5: 3-Chlor-N-isopropyl-anilin

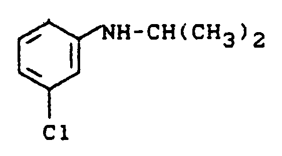

In einem 1,3-l-Rührautoklaven wurden 116 g Aceton, 255 g 3-Chloranilin, 500 ml Methanol, 20 g Raney-Nickel und 2 g Bis-(2-hydroxyethyl)-sulfid vorgelegt. Nach Verdrängen der Luft mit Stickstoff wurde Wasserstoff bis zu einem Druck von 80 bar aufgedrückt und unter Rühren auf 120°C geheizt. Durch weitere Zufuhr von Wasserstoff wurde der Druck auf 150 bar eingestellt und der Verbrauch kompensiert. Die Temperatur wurde noch bis 1 Stunde nach dem Ende der H₂-Aufnahme bei 120°C gehalten.

Nach dem Abkühlen und Entspannen wurde der Katalysator abfiltriert und die Reaktionslösung eingedampft.

Die so gewonnenen 308 g Rohprodukt enthielten nach gaschromatographischer Analyse:
77% 3-Chlor-N-isopropyl-anilin
12% N-Isopropyl-anilin
7% 3-Chloranilin
4% nicht identifizierte Nebenprodukte.

### Beispiel 6: 4-Chlor-N-isopropyl-anilin

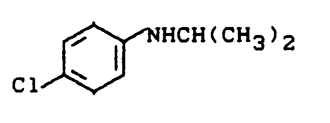

In einem 1,3-l-Rührautoklaven wurden 232 g Aceton, 255 g 4-Chloranilin, 300 ml Ethanol, 25 g Raney-Nickel-Eisen und 2,5 g Bis-(2-hydroxyethyl)-sulfid vorgelegt. Nach Verdrängen der Luft mit Stickstoff wurde Wasserstoff bis zu einem Druck von 60 bar aufgedrückt und unter Rühren auf 100°C geheizt. Durch weitere Zufuhr von Wasserstoff wurde der Druck auf 100 bar eingestellt und der Verbrauch kompensiert. Die Temperatur wurde noch bis 1 Stunde nach dem Ende der H₂-Aufnahme bei 100°C gehalten.

Nach dem Abkühlen und Entspannen wurde der Katalysator abfiltriert und die Reaktionslösung eingedampft.

Die so gewonnenen 310 g Rohprodukt enthielten nach gaschromatographischer Analyse:
82% 4-Chlor-N-isopropyl-anilin
8,5% N-Isopropyl-anilin
6,5% 4-Chloranilin und
3% nicht identifizierte Nebenprodukte.

### Beispiel 7: 3-Chlor-N-neopentyl-anilin

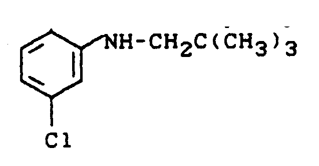

In einem 1,3-l-Rührautoklaven wurden 172 g Pivalaldehyd, 255 g 3-Chloranilin, 400 ml Tetrahydrofuran, 25 g Raney-Nickel und 2,5 g Bis-(2-hydroxyethyl)-sulfid vorgelegt. Nach Verdrängen der Luft mit Stickstoff wurde Wasserstoff bis zu einem Druck von 80 bar aufgedrückt und unter Rühren auf 120°C geheizt. Durch weitere Zufuhr von Wasserstoff wurde der Druck auf 140 bar eingestellt und der Verbrauch kompensiert. Die Temperatur wurde noch bis 1 Stunde nach dem Ende der H₂-Aufnahme bei 120°C gehalten.

Nach dem Abkühlen und Entspannen wurde der Katalysator abfiltriert und die Reaktionslösung eingedampft.

Die so gewonnenen 355 g Rohprodukt enthielten nach gaschromatographischer Analyse:
76% 3-Chlor-N-neopentyl-anilin
7,5% N-Neopentyl-anilin
12,5% 3-Chloranilin und
4% nicht identifizierte Nebenprodukte.

### Beispiel 8: 3-Amino-5-(4-Chlorphenyl)-2,2-dimethylpentan

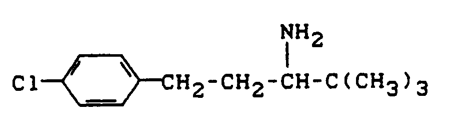

In einem 1,3-l-Rührautoklaven wurden 168 g 5-(4-Chlorphenyl)-2,2-dimethyl-pentan-3-on, 160 ml Methanol, 20 g Raney-Nickel, 1 g Bis-(2-hydroxyethyl)-sulfid und 5 g Ammoniumacetat vorgelegt. Nach Verdrängen der Luft mit Stickstoff und Zugabe von 500 ml flüssigem Ammoniak wurde Wasserstoff bis zu einem Druck von 90 bar aufgedrückt und unter Rühren auf 125°C geheizt. Durch weitere Zufuhr von Wasserstoff wurde der Druck auf 120 bar eingestellt und der Verbrauch kompensiert. Die Temperatur wurde noch bis 1 Stunde nach dem Ende der H₂-Aufnahme bei 125°C gehalten.

Nach dem Abkühlen und Entspannen wurde der Katalysator abfiltriert und die Reaktionslösung eingedampft.

Die so gewonnenen 172 g Rohprodukt enthielten nach gaschromatographischer Analyse:
73% 3-Amino-5-(4-Chlorphenyl)-2,2-dimethyl-pentan
5% 5-(4-Chlorphenyl)-2,2-dimethyl-3-hydroxy-pentan
0,5% dechlorierte Verbindungen
20% Edukt.

### Beispiel 9: 2-Amino-4-chlor-benzylamin

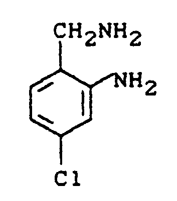

In einem 1,3-l-Rührautoklaven wurden 152 g (1 Mol) 2-Amino-4-Chlor-benzonitril, 400 ml Isopropanol, 15 g Raney Nickel und 1 g Bis-(2-hydroxyethyl)-sulfid vorgelegt. Nach Verdrängen der Luft mit Stickstoff und Zugabe von 150 ml flüssigem Ammoniak wurde Wasserstoff bis zu einem Druck von 80 bar aufgedrückt und unter Rühren auf 80°C geheizt. Durch weitere Zufuhr von Wasserstoff wurde der Druck auf 140 bar eingestellt und der Verbrauch kompensiert. Die Temperatur wurde noch bis 1 Stunde nach dem Ende der H₂-Aufnahme bei 80°C gehalten.

Nach dem Abkühlen und Entspannen wurde der Katalysator abfiltriert und die Reaktionslösung eingedampft.

Die so gewonnenen 153 g Rohprodukt enthielten nach gaschromatographischer und massenspektroskopischer (GC-MS-Kopplung) Analyse:
98.5% 2-Amino-4-chlor-benzylamin und
<0,1% Dechlorierungsprodukte.

### Beispiel 10: 4-Amino-2,5-dichlor-benzylamin

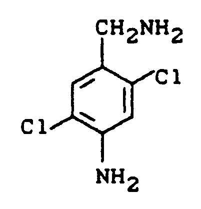

In einem 1,3-l-Rührautoklaven wurden 94 g (0,5 Mol) 4-Amino-2,5-dichlor-benzonitril, 300 ml Methanol, 10 g Raney-Nickel-Eisen und 1 g Bis-(2-hydroxyethyl)-sulfid vorgelegt. Nach Verdrängen der Luft mit Stickstoff und Zugabe von 200 ml flüssigem Ammoniak wurde Wasserstoff bis zu einem Druck von 60 bar aufgedrückt und unter Rühren auf 70°C geheizt. Durch weitere Zufuhr von Wasserstoff wurde der Druck auf 100 bar eingestellt und der Verbrauch kompensiert. Die Temperatur wurde noch bis 1 Stunde nach dem Ende der H₂-Aufnahme bei 70°C gehalten.

Nach dem Abkühlen und Entspannen wurde der Katalysator abfiltriert und die Reaktionslösung eingedampft.

Die so gewonnenen 95 g Rohprodukt enthielten nach gaschromatographischer und MS-Analyse:
95% 4-Amino-2,5-dichlor-benzylamin.

### Beispiel 11: 4-Chlorphenyl-phenyl-methylamin

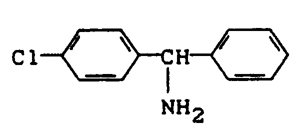

In einem 1,3-l-Rührautoklaven wurden 162 g 4-Chlorbenzophenon, 450 ml Methanol, 10 g Raney-Nickel, 1,5 g Bis-(2-hydroxyethyl)-sulfid und 5 g Ammoniumacetat vorgelegt. Nach Verdrängen der Luft mit Stickstoff und Zugabe von 300 ml flüssigem Ammoniak wurde Wasserstoff bis zu einem Druck von 80 bar aufgedrückt und unter Rühren auf 125°C geheizt. Durch weitere Zufuhr von Wasserstoff wurde der Druck auf 120 bar eingestellt und der Verbrauch kompensiert. Die Temperatur wurde noch bis 1 Stunde nach dem Ende der H₂-Aufnahme bei 125°C gehalten.

Nach dem Abkühlen und Entspannen wurde der Katalysator abfiltriert und die Reaktionslösung eingedampft.

Die so gewonnenen 160 g Rohprodukt enthielten nach gaschromatographischer und massenspektroskopischer Analyse:
88 % (4-Chlorphenyl)-phenyl-methyl-amin
3 % Diphenyl-methylamin
2,4% (4-Chlorphenyl)-phenyl-methan
3,4% Bis-[(4-chlorphenyl)-phenylmethyl]amin
1,3% Edukt

### Beispiel 12: 1-Amino-2-(4-Chlorphenyl)-ethan

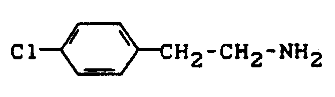

In einem 0,7-l-Rührautoklaven wurden 100 g 4-Chlorbenzylcyanid, 100 g Methanol, 5,0 g Raney-Nickel wasserfeucht und 0,75 g Bis-(2-hydroxyethyl)-sulfid vorgelegt. Nach Verdrängen der Luft mit Stickstoff und Zugabe von 30 g Ammoniak wurde Wasserstoff bis zu einem Druck von 100 bar aufgedrückt und unter Rühren auf 130°C geheizt. Sobald eine Wasserstoffaufnahme zu erkennen war, wurde durch weitere Zufuhr von Wasserstoff der Druck auf 150 bar eingestellt und der Verbrauch kompensiert. Die Temperatur wurde noch bis 30 Minuten nach dem Ende der H₂-Aufnahme bei 130°C gehalten.

Nach dem Abkühlen und Entspannen wurde der Katalysator abfiltriert. Die Reaktionslösung enthielt nach gaschromatographischer Analyse:
0,77 % unbekannte Substanzen (4 Peaks)
99,01 % 1-Amino-2-(p-chlorphenyl)ethan
0,22 % unbekannte Substanzen (3 Peaks)

### Beispiel 13 (zum Vergleich):

Es wurde wie im Beispiel 12, jedoch ohne Bis-(2-hydroxyethyl)-sulfid gearbeitet. Die gaschromatographische Analyse ergab:
0,71 % unbekannte Substanzen (4 Peaks)
91,67 % 1-Amino-2-(p-chlorphenyl)ethan
0,03 % unbekannte Substanz
1,96 % unbekannte Substanz
5,58 % unbekannte Substanz
0,05 % unbekannte Substanz (insgesamt 4 Peaks)

### Beispiel 14 o-Chlorbenzylamin:

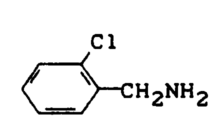

In einem 0,7-l-Rührautoklaven wurden 140,6 g o-Chlorbenzaldehyd, 100 g Methanol, 8,4 g Raney-Nickel methanolfeucht, und 0,2 g Bis-(2-hydroxyethyl)-sulfid vorgelegt. Nach Verdrängen der Luft mit Stickstoff und Zugabe von 102 g Ammoniak wurde Wasserstoff bis zu einem Druck von 90 bar aufgedrückt und unter Rühren auf 100°C geheizt. Durch weitere Zufuhr von Wasserstoff wurde der Druck immer wieder auf 90 bar eingestellt und so der Verbrauch kompensiert. Die Temperatur wurde noch bis 1 Stunde nach dem Ende der H₂-Aufnahme bei 100°C gehalten.

Nach dem Abkühlen und Entspannen wurde der Katalysator abfiltriert. Die Reaktionslösung enthielt nach gaschromatographischer Analyse:
0,9 % Benzylamin
90,5 % o-Chlorbenzylamin
6,8 % o-Chlorbenzylalkohol

### Beispiel 15 (zum Vergleich):

Es wurde wie im Beispiel 14, jedoch ohne Bis-(2-hydroxyethyl)-sulfid gearbeitet. Die gaschromatographische Analyse ergab:
3,6 % Benzylamin
80,2 % o-Chlorbenzylamin
13,8 % o-Chlorbenzylalkohol

### Beispiel 16 α-(p-Chlorphenyl)-N,N-dimethyl-ethylamin

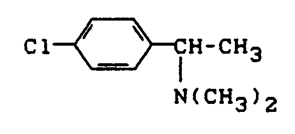

In einem 0,3-l-Rührautoklaven wurden 15,5 g (0,1 Mol) α-(p-Chlorphenyl)-ethylamin, 100 ml Methanol, 13,5 g einer 50 %igen methanolischen Formaldehydlösung (0,22 Mol), 2 g Raney-Nickel und 0,15 g Bis-(2-hydroxyethyl)-sulfid vorgelegt. Nach Verdrängen der Luft mit Stickstoff wurde Wasserstoff bis zu einem Druck von 80 bar aufgedrückt und unter Rühren auf 110°C geheizt. Durch weitere Zufuhr von Wasserstoff wurde der Druck auf 160 bar eingestellt und der Verbrauch kompensiert. Die Temperatur wurde noch bis 1 Stunde nach dem Ende der H₂-Aufnahme bei 110°C gehalten.

Nach dem Abkühlen und Entspannen wurde der Katalysator abfiltriert und die Reaktionslösung eingedampft.

Die so gewonnenen 16,9 Rohprodukt enthielten nach gaschromatographischer und massenspektroskopischer Analyse:
91,8 % α-(p-Chlorphenyl)-N,N-dimethyl-ethylamin,
1,5 % α-(p-Chlorphenyl)-N-methyl-ethylamin,
5,7 % N,N-Dimethyl-α-phenyl-ethylamin und
1 % nicht identifizierte Nebenprodukte.

### Beispiel 17 α-(3,4-Dichlorphenyl)-ethylamin

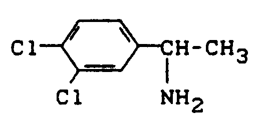

In einem 0,7 l-Rührautoklaven wurden 94,5 g 3,4-Dichloracetophenon, 225 ml Methanol, 2,5 g Ammoniumacetat, 5 g Raney-Nickel und 1 g Bis-(2-hydroxyethyl)-sulfid vorgelegt. Nach Verdrängen der Luft mit Stickstoff und Zugabe von 150 ml flüssigem Ammoniak wurde Wasserstoff bis zu einem Druck von 80 bar aufgedrückt und unter Rühren auf 125°C geheizt. Durch weitere Zufuhr von Wasserstoff wurde der Druck auf 120 bar eingestellt und der Verbrauch kompensiert. Die Temperatur wurde noch bis 1 Stunde nach dem Ende der H₂-Aufnahme bei 125°C gehalten.

Nach dem Abkühlen und Entspannen wurde der Katalysator abfiltriert und die Reaktionslosung eingedampft.

Die so gewonnenen 93 g Rohprodukt enthielten nach gaschromatographischer Analyse;
87 % α-(3,4-Dichlorphenyl)-ethylamin,
3 % α-Phenethylamin,
4,5 % α-(3,4-Dichlorphenyl)-ethanol und
5,5 % nicht identifizierte Nebenprodukte.

## Patentansprüche

1. Verfahren zur Herstellung von Aminen der Formel 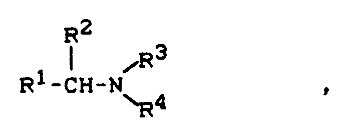 in der
R¹, R² und R³ unabhängig voneinander Wasserstoff, geradkettiges oder verzweigtes C₁-C₁₂-Alkyl, C₃-C₈-Cycloalkyl, C₆-C₁₂-Aryl oder C₇-C₁₀-Aralkyl bedeuten und
R⁴ für Wasserstoff, geradkettiges oder verzweigtes C₁-C₂₀-Alkyl, C₆-C₁₂-Aryl, C₃-C₈-Cycloalkyl oder C₇-C₁₀-Aralkyl steht,
wobei mindestens einer der Reste R¹ bis R⁴ Aryl oder Aralkyl darstellt, die im aromatischen Teil ein- bis dreifach durch Halogen substituiert sind,
durch katalytische Hydrierung von Umsetzungsprodukten aus Oxoverbindungen der Formel
R¹-CO-R²
und Stickstoffverbindungen der Formel
R³-NH-R⁴
mit den obengenannten Bedeutungen für R¹ bis R⁴,
dadurch gekennzeichnet, daß ein Ni-haltiger und/oder Co-haltiger Katalysator eingesetzt wird und in Gegenwart von organischen Schwefelverbindungen der Formel
R⁵-S(=O)ₙ-R⁶
gearbeitet wird, in der
R⁵ und R⁶ unabhängig voneinander geradkettiges oder verzweigtes C₁-C₁₂-Alkyl, Hydroxy-C₂-C₁₂-alkyl, Carboxy-C₁-C₁₂-alkyl oder Phenyl bedeuten und weiterhin
R⁵ und R⁶ gemeinsam für -CH=CH-CH=CH-, -(CH₂)₄-, -(CH₂)₅-, -(CH₂)₂-S-(CH₂)₂- oder -(CH₂)₂-O-(CH₂)₂-stehen können,
R⁶ zusätzlich Wasserstoff oder CO-C₁-C₁₂-alkyl bedeuten kann und
n den Wert 0 oder 1 annimmt.

2. Verfahren nach Anspruch 1, dadurch gekennzeichnet, daß organische Schwefelverbindungen der Formel
R¹⁵-S(=O)ₙ-R¹⁶
eingesetzt werden, in der
R¹⁵ und R¹⁶ unabhängig voneinander geradkettiges oder verzweigtes C₂-C₁₂-Alkyl, Hydroxy-C₂-C₁₂-alkyl oder Carboxy-C₁-C₁₂-alkyl bedeuten und wobei
R¹⁶ zusätzlich CO-C₁-C₆-alkyl bedeuten kann und
n den Wert 0 oder 1 annimmt.

3. Verfahren nach Anspruch 2, dadurch gekennzeichnet, daß Bis-(2-hydroxyethyl)-sulfid eingesetzt wird.

4. Verfahren nach Anspruch 1, dadurch gekennzeichnet, daß als Katalysator Raney-Nickel, Raney-Kobalt, Raney-Nickel-Eisen, Raney-Nickel-Kobalt oder Raney-Nickel-Eisen-Kobalt eingesetzt wird.

5. Verfahren nach Anspruch 1, dadurch gekennzeichnet, daß Reaktionsgemische, in denen die Umsetzungsprodukte der Oxoverbindungen und der Stickstoffverbindungen hergestellt werden, eingesetzt werden.

6. Verfahren nach Anspruch 1, dadurch gekennzeichnet, daß Reaktionsgemische eingesetzt werden, in denen Azomethine der Formel
R³¹-CH=NH,
in der
R¹ C₁-C₁₂-Alkyl, C₃-C₈-Cycloalkyl, C₆-C₁₂-Aryl oder C₇-C₁₀-Aralkyl bedeutet,
durch partielle Hydrierung aus den zugrundeliegenden Nitrilen hergestellt werden.

7. Verfahren nach Anspruch 5, dadurch gekennzeichnet, daß als Oxoverbindungen solche der Formel
R¹¹-CO-R¹²
eingesetzt werden, in der
R¹¹ Wasserstoff, geradkettiges oder verzweigtes C₁-C₁₂-Alkyl, C₃-C₆-Cycloalkyl, Phenyl, Benzyl oder Phenyl-ethyl bedeutet und
R¹² für Wasserstoff, geradkettiges oder verzweigtes C₁-C₄-Alkyl oder Phenyl steht.

8. Verfahren nach Anspruch 7, dadurch gekennzeichnet, daß als Oxoverbindungen solche der Formel
R²¹-CO-R²²
eingesetzt werden, in der
R²¹ Wasserstoff, geradkettiges oder verzweigtes C₁-C₄-Alkyl, C₅-C₆-Cycloalkyl, Phenyl, Benzyl oder Phenyl-ethyl bedeutet und
R²² für Wasserstoff, Methyl oder Ethyl steht.

9. Verfahren nach Anspruch 1, dadurch gekennzeichnet, daß als Stickstoffverbindungen solche der Formel
R¹³-NH-R¹⁴
eingesetzt werden, in der
R¹³ Wasserstoff, geradkettiges oder verzweigtes C₁-C₈-Alkyl, C₃-C₆-Cycloalkyl, Phenyl oder Benzyl bedeutet und
R¹⁴ für Wasserstoff, geradkettiges oder verzweigtes C₁-C₄-Alkyl oder Benzyl steht.

10. Verfahren nach Anspruch 9, dadurch gekennzeichnet, daß man als Stickstoffverbindungen solche der Formel
R²³-NH-R²⁴
einsetzt, in der
R²³ Wasserstoff, geradkettiges oder verzweigtes C₁-C₄-Alkyl, C₅-C₆-Cycloalkyl, Phenyl oder Benzyl bedeutet und
R²⁴ für Wasserstoff, Methyl oder Ethyl steht.

## Claims

1. Process for the preparation of amines of the formula 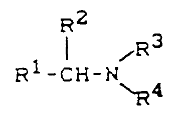 in which
R¹, R² and R³ independently of one another denote hydrogen, straight-chain or branched C₁-C₁₂-alkyl, C₃-C₈-cycloalkyl, C₆-C₁₂-aryl or C₇-C₁₀-aralkyl and
R⁴ represents hydrogen, straight-chain or branched C₁-C₂₀-alkyl, C₆-C₁₂-aryl, C₃-C₈-cycloalkyl or C₇-C₁₀-aralkyl,
where at least one of the radicals R¹ to R⁴ represents aryl or aralkyl which are monosubstituted to trisubstituted by halogen in the aromatic moiety,
by catalytic hydrogenation of reaction products of oxo compounds of the formula
R¹-CO-R²
and nitrogen compounds of the formula
R³-NH-R⁴
with the abovementioned meanings for R¹ to R⁴,
characterized in that an Ni-containing and/or Co-containing catalyst is employed and the reaction is carried out in the presence of organic sulphur compounds of the formula
R⁵-S(=O)ₙ-R⁶
in which
R⁵ and R⁶ independently of one another denote straight-chain or branched C₁-C₁₂-alkyl, hydroxy-C₂-C₁₂-alkyl, carboxy-C₁-C₁₂-alkyl or phenyl and furthermore
R⁵ and R⁶ together may represent -CH=CH-CH=CH-, -(CH₂)₄-, -(CH₂)₅-, -(CH₂)₂-S-(CH₂)₂- or -(CH₂)₂-O-(CH₂)₂-,
R⁶ additionally may denote hydrogen or CO-C₁-C₁₂-alkyl and
n assumes the value 0 or 1.

2. Process according to Claim 1, characterized in that organic sulphur compounds of the formula
R¹⁵-S(=O)ₙ-R¹⁶
are employed in which
R¹⁵ and R¹⁶ independently of one another denote straight-chain or branched C₂-C₁₂-alkyl, hydroxy-C₂-C₁₂-alkyl or carboxy-C₁-C₁₂-alkyl and where
R¹⁶ additionally may denote CO-C₁-C₆-alkyl and
n assumes the value 0 or 1.

3. Process according to Claim 2, characterized in that bis-(2-hydroxyethyl) sulphide is employed.

4. Process according to Claim 1, characterized in that Raney nickel, Raney cobalt, Raney nickel-iron, Raney nickel-cobalt or Raney nickel-iron-cobalt are employed as catalyst.

5. Process according to Claim 1, characterized in that reaction mixtures in which the reaction products of the oxo compounds and the nitrogen compounds are prepared are employed.

6. Process according to Claim 1, characterized in that reaction mixtures are employed in which azomethines of the formula
R³¹-CH=NH
in which
R¹ denotes C₁-C₁₂-alkyl, C₃-C₈-cycloalkyl, C₆-C₁₂-aryl or C₇-C₁₀-aralkyl,
are prepared by partial hydrogenation of the underlying nitriles.

7. Process according to Claim 5, characterized in that, as oxo compounds, those of the formula
R¹¹-CO-R¹²
are employed in which
R¹¹ denotes hydrogen, straight-chain or branched C₁-C₁₂-alkyl, C₃-C₆-cycloalkyl, phenyl, benzyl or phenyl-ethyl and
R¹² represents hydrogen, straight-chain or branched C₁-C₄-alkyl or phenyl.

8. Process according to Claim 7, characterized in that, as oxo compounds, those of the formula
R²¹-CO-R²²
are employed in which
R²¹ denotes hydrogen, straight-chain or branched C₁-C₄-alkyl, C₅-C₆-cycloalkyl, phenyl, benzyl or phenyl-ethyl and
R²² represents hydrogen, methyl or ethyl.

9. Process according to Claim 1, characterized in that, as nitrogen compounds, those of the formula
R¹³-NH-R¹⁴
are employed in which
R¹³ denotes hydrogen, straight-chain or branched C₁-C₈-alkyl, C₃-C₆-cycloalkyl, phenyl or benzyl and
R¹⁴ represents hydrogen, straight-chain or branched C₁-C₄-alkyl or benzyl.

10. Process according to Claim 9, characterized in that, as nitrogen compounds, those of the formula
R²³-NH-R²⁴
are employed in which
R²³ denotes hydrogen, straight-chain or branched C₁-C₄-alkyl, C₅-C₆-cycloalkyl, phenyl or benzyl and
R²⁴ represents hydrogen, methyl or ethyl.

## Revendications

1. Procédé de préparation d'amines de formule 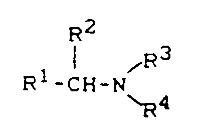 dans laquelle
R¹, R² et R³ représentent chacun, indépendamment les uns des autres, l'hydrogène, un coupe alkyle à chaîne droite ou ramifiée en C₁-C₁₂, un groupe cycloalkyle en C₃-C₈, aryle en C₆-C₁₂ ou arylalkyle en C₇-C₁₀ et
R⁴ représente l'hydrogène, un groupe alkyle à chaîne droite ou ramifiée en C₁-C₂₀, un groupe aryle en C₆-C₁₂, cycloalkyle en C₃-C₈ ou aralkyle en C₇-C₁₀, l'un au moins des symboles R¹ à R⁴ représentant un groupe aryle ou aralkyle qui est mono- à tri-substitué dans la partie aromatique par des halogènes,
par hydrogénation catalytique de produits de réaction de dérivés en oxo de formule
R¹-CO-R²
et de dérivés azotés de formule
R³-NH-R⁴
dans lesquelles R¹ à R⁴ ont les significations indiquées ci-dessus,
caractérisé en ce que l'on utilise un catalyseur contenant du nickel et/ou du cobalt et on opère en présence de dérivés organiques du soufre de formule
R⁵-S(=O)ₙ-R⁶
dans laquelle
R⁵ et R⁶ représentent chacun, indépendamment l'un de l'autre, un groupe alkyle à chaîne droite ou ramifiée en C₁-C₁₂, hydroxyalkyle en C₂-C₁₂, carboxy-alkyle en C₁-C₁₂ ou phényle,
R⁵ et R⁶ pouvant également former ensemble un groupe -CH=CH-CH=CH-, -(CH₂)₄-, -(CH₂)₅-, -(CH₂)₂-S-(CH₂)₂- ou -(CH₂)₂-O-(CH₂)₂-,
R⁶ peut en outre représenter l'hydrogène ou un groupe CO-alkyle en C₁-C₁₂ et
n est égal à 0 ou 1.

2. Procédé selon la revendication 1, caractérisé en ce que l'on utilise des dérivés organiques du soufre répondant à la formule
R¹⁵-S(=O)ₙ-R¹⁶
dans laquelle
R¹⁵ et R¹⁶ représentent chacun, indépendamment l'un de l'autre, un groupe alkyle à chaîne droite ou ramifiée en C₂-C₁₂, hydroxyalkyle en C₂-C₁₂ ou carboxy-alkyle en C₁-C₁₂,
R¹⁶ pouvant en outre représenter un groupe CO-alkyle en C₁-C₆ et
n est égal à 0 ou 1.

3. Procédé selon la revendication 2, caractérisé en ce que l'on utilise le sulfure de bis-(2-hydroxyéthyle).

4. Procédé selon la revendication 1, caractérisé en ce que l'on utilise en tant que catalyseur du nickel de Raney, du cobalt de Raney, du nickel-fer de Raney, du nickel-cobalt de Raney ou du nickel-fer-cobalt de Raney.

5. Procédé selon la revendication 1, caractérisé en ce que l'on met en oeuvre les mélanges de réaction dans lesquels on a préparé les produits de réaction des dérivés en oxo et des dérivés azotés.

6. Procédé selon la revendication 1, caractérisé en ce que l'on met en oeuvre des mélanges de réaction dans lesquels on a préparé par hydrogénation partielle, à partir des nitriles correspondants, des azométhines de formule
R³¹-CH=NH
dans laquelle
R¹ représente un groupe alkyle en C₁-C₁₂, cycloalkyle en C₃-C₈, aryle en C₆-C₁₂ ou aralkyle en C₇-C₁₀.

7. Procédé selon la revendication 5, caractérisé en ce que l'on utilise des dérivés en oxo répondant à la formule
R¹¹-CO-R¹²
dans laquelle
R¹¹ représente l'hydrogène, un groupe alkyle à chaîne droite ou ramifiée en C₁-C₁₂, cycloalkyle en C₃-C₆, phényle, benzyle ou phényléthyle et
R¹² représente l'hydrogène, un groupe alkyle à chaîne droite ou ramifiée, en C₁-C₄ ou un groupe phényle.

8. Procédé selon la revendication 7, caractérisé en ce que l'on utilise des dérivés en oxo répondant à la formule
C²¹-CO-R²²
dans laquelle
R²¹ représente l'hydrogène, un groupe alkyle à chaîne droite ou ramifiée en C₁-C₄, un groupe cycloalkyle en C₅-C₆, phényle, benzyle ou phényléthyle et R²² représente l'hydrogène, un groupe méthyle ou éthyle.

9. Procédé selon la revendication 1, caractérisé en ce que l'on utilise des dérivés azotés répondant à la formule
R¹³-NH-R¹⁴
dans laquelle
R¹³ représente l'hydrogène, un groupe alkyle à chaîne droite ou ramifiée en C₁-C₈, un groupe cycloalkyle en C₃-C₆, phényle ou benzyle et
R¹⁴ représente l'hydrogène, un groupe alkyle à chaîne droite ou ramifiée en C₁-C₄ ou un groupe benzyle.

10. Procédé selon la revendication 9, caractérisé en ce que l'on utilise des dérivés azotés répondant à la formule
R²³-NH-R²⁴
dans laquelle
R²³ représente l'hydrogène, un groupe alkyle à chaîne droite ou ramifiée en C₁-C₄, un groupe cycloalkyle en C₅-C₆, phényle ou benzyle et
R²⁴ représente l'hydrogène, un groupe méthyle ou éthyle.
